# EUROPEAN PATENT APPLICATION

(11) **EP 4 403 036 A1**
(43) Date of publication of application: **24.07.2024**
(21) Application number: 22869970.8
(22) Date of filing: 13.09.2022
(51) Int. Cl.: A23F 5/02, A23F 5/10

(54) **COFFEE BEANS AND METHOD FOR PRODUCING COFFEE BEANS**

(30) Priority: 14.09.2021 JP 2021149175
(71) Applicant: Suntory Holdings Limited, Osaka 530-8203 (JP)
(72) Inventor: SUGINO, Ryosuke, Kawasaki-shi, Kanagawa 211-0067 (JP); KATAYAMA, Makoto, Kawasaki-shi, Kanagawa 211-0067 (JP)
(74) Representative: Mewburn Ellis LLP
(86) International application number: PCT/JP2022/034232
(87) International publication number: WO 2023/042823

(57) **Abstract**

The present invention provides: coffee beans richer in 2-phenylethanol, which has an influence on coffee flavor, than conventional ones; and a method for producing such coffee beans. An aspect of the present invention provides coffee beans containing at least 9 ppm of 2-phenylethanol. Another aspect of the present invention provides a method for producing coffee beans containing at least 9 ppm of 2-phenylethanol, comprising a step (i) of adding to raw material beans a yeast, and at least 0.01 g of phenylalanine per 1 kg of the raw material beans, in which the raw material beans are coffee cherries, wet parchment obtained by removing outer skin and pulp of coffee cherries, unroasted coffee beans, or a combination of at least two thereof; and a step (ii) of fermenting the raw material beans with the yeast and phenylalanine added.

## Description

### Technical Field

The present invention relates to coffee beans and a method for producing the coffee beans.

### Background Art

The term "coffee beans" collectively refers to coffee seeds and the beans processed from coffee seeds. Coffee seeds are obtained by a process called refining, during which the pulp and the thin skin are removed from the berries of Coffea plants of the family Rubiaceae, known as coffee berries or coffee cherries. Of these, unroasted coffee beans are coffee beans that have not yet been subjected to roasting, which is a process of heating and roasting coffee beans, and roasted coffee beans are coffee beans that have been subjected to roasting.

There are unroasted coffee beans containing aroma components imparted by fermentation. Patent Document 1 discloses modified unroasted coffee beans having 2 to 3 times as much amount of phenylethyl alcohol component as that of standard coffee beans. The relative area of phenylethyl alcohol obtained by performing GC/MS analysis on the modified unroasted coffee beans is from 0.11 to 0.25 with respect to the relative area of 1.00 of ethyl hexanoate, which is a standard substance. The technique of Patent Document 1 imparts a value-added flavor to a coffee extract liquid without the acquisition and usage of yeast or microorganisms.

Patent Document 2 discloses a technique of improving aroma by adding nutritive substances and yeast to coffee beans and subjecting the mixture to fermentation. Non-Patent Document 1 discloses the separation and production mechanism of a yeast producing β-phenylethanol in sake brewing, and discloses that the yeast produces β-phenylethanol (2-phenylethanol) from phenylalanine using the Ehrlich pathway.

### Citation List

### Patent Document

Patent Document 1: JP 2018-50535 A
Patent Document 2: JP 5032979 B

### Non-Patent Literature

Non-Patent Document 1: Kazuyoshi Koganemaru et al., J. Brew. Soc. Japan. Vol. 98, No. 3, pp. 201 to 209 (2003)

### Summary of Invention

### Technical Problem

Under such circumstances, the provision of coffee beans that can yield coffee having a rich aroma is long-awaited.

### Solution to Problem

The present invention provides coffee beans and a method for producing the coffee beans as described below.
[1] Coffee beans containing 2-phenylethanol in an amount of 9 ppm or greater.
[2] The coffee beans according to [1], in which the coffee beans contain the 2-phenylethanol in an amount from 9 ppm to 2000 ppm.
[3] The coffee beans according to [1] or [2], in which the coffee beans are unroasted coffee beans or roasted coffee beans.
[4] The coffee beans according to any one of [1] to [3], in which the content of 2-phenylethanol in the coffee beans is measured according to the following measurement method.
   [Measurement Method]
      1 g of coffee beans is placed in a test tube, 20 mL of water and 10 mL of diethyl ether are added, and the coffee beans are allowed to soak for 1 hour. Afterwards, 8 g of sodium chloride is added, and the mixture is shaken and centrifuged. 1 mL of the resulting diethyl ether layer is taken, to which diethyl ether is further added to make 20 mL of a liquid. The content of 2-phenylethanol in the resulting liquid is measured by gas chromatography-mass spectrometry using the following gas chromatography-mass spectrometer.
   [Gas Chromatography-Mass Spectrometer]
      Model: 6890A/5973N [Agilent Technologies, Inc.]
      Column: DB-WAX UI (ϕ 0.25 mm × 60 m, film thickness 0.5 µm) [Agilent Technologies, Inc.]
      Injection method: Split 5:1
      Temperature: 220°C at sample inlet
      Column: Maintain at 60°C for 1 minute, increase temperature at 10°C/min, maintain at 240°C for 5 minutes
      Gas flow rate: helium (carrier gas) 1 mL/min
      Ion source temperature: 230°C
      Ionization method: EI
      Set mass number: m/z 91.
[5] A method for producing coffee beans containing 2-phenylethanol in an amount of 9 ppm or greater, the method including
   (i) adding, to raw material beans, a yeast, and phenylalanine in an amount of 0.01 g or greater per 1 kg of the raw material beans, in which the raw material beans are coffee cherries, wet parchment with outer skin and pulp of coffee cherries removed, unroasted coffee beans, or a combination of two or more thereof; and
   (ii) fermenting the raw material beans with the yeast and phenylalanine added.
[6] The production method according to [5], in which the yeast is selected from the group consisting of Candida sake, Brettanomyces sp., Zygosaccharomyces bisporus, Saccharomyces cerevisiae, and a combination of two or more of the foregoing.
[7] The production method according to [5] or [6], in which in the step (ii), the raw material beans are fermented for 1 hour or longer.
[8] The method for producing coffee beans according to any one of [5] to [7], in which the content of 2-phenylethanol in the coffee beans is measured according to the following measurement method.
   [Measurement Method]
      1 g of coffee beans is placed in a test tube, 20 mL of water and 10 mL of diethyl ether are added, and the coffee beans are allowed to soak for 1 hour. Afterwards, 8 g of sodium chloride is added, and the mixture is shaken and centrifuged. 1 mL of the resulting diethyl ether layer is taken, to which diethyl ether is further added to make 20 mL of a liquid. The content of 2-phenylethanol in the resulting liquid is measured by gas chromatography-mass spectrometry using the following gas chromatography-mass spectrometer.
   [Gas Chromatography-Mass Spectrometer]
      Model: 6890A/5973N [Agilent Technologies, Inc.]
      Column: DB-WAX UI (ϕ 0.25 mm × 60 m, film thickness 0.5 µm) [Agilent Technologies, Inc.]
      Injection method: Split 5:1
      Temperature: 220°C at sample inlet
      Column: Maintain at 60°C for 1 minute, increase temperature at 10°C/min, maintain at 240°C for 5 minutes
      Gas flow rate: helium (carrier gas) 1 mL/min
      Ion source temperature: 230°C
      Ionization method: EI
      Set mass number: m/z 91.

### Advantageous Effects of Invention

The present invention allows for the provision of coffee beans that can yield coffee having a rich aroma.

The present invention allows for the provision of a method for producing coffee beans using coffee cherries, wet parchment, and/or unroasted coffee beans as raw material beans in coffee-producing countries.

The present invention allows for the provision of a method for producing coffee beans using unroasted coffee beans as raw material beans in coffee-consuming countries.

The present invention allows for the provision of a method for producing coffee beans that can be carried out during the transportation of coffee cherries, wet parchment, and/or unroasted coffee beans.

The coffee beans according to an aspect of the present invention contains 2-phenylethanol in an amount of 9 ppm or greater, which is about 4.5 or more times as much amount of 2-phenylethanol as that in commercially available coffee beans (Comparative Examples 11 to 19 described below), allowing for the provision of coffee having a rich aroma.

### Description of Embodiments

### Coffee Beans

The coffee beans according to an embodiment of the present invention are unroasted coffee beans or roasted coffee beans. The coffee beans may include both unroasted coffee beans and roasted coffee beans.

Unroasted coffee beans are seeds contained in coffee berries (coffee cherries). Inside, a coffee cherry contains a seed, which is covered in turn by a sticky substance (mucilage), pulp (a part that contains sugars and other nutrients), and outer skin. Unroasted coffee beans may be undried or dried. In addition, unroasted coffee beans may be whole beans or at least partially ground beans.

Roasted coffee beans are obtained by roasting coffee beans. Roasted coffee beans may be whole beans or may be a ground powder.

The coffee beans according to an aspect of the present invention contain 2-phenylethanol in an amount of 9 ppm or greater. The unit "ppm" is the ratio (mg/kg) of the mass (mg) of 2-phenylethanol contained in 1 kg of coffee beans.

The unroasted coffee beans according to an aspect of the present invention contain 2-phenylethanol in an amount of 9 ppm or greater. In addition, the roasted coffee beans according to an aspect of the present invention contain 2-phenylethanol in an amount of 9 ppm or greater.

In addition to 2-phenylethanol, the coffee beans may contain 1-phenylethanol as a phenylethanol component.

The content of 2-phenylethanol contained in the coffee beans may be 15 ppm or greater, 20 ppm or greater, 30 ppm or greater, 40 ppm or greater, 50 ppm or greater, 100 ppm or greater, 150 ppm or greater, 200 ppm or greater, 250 ppm or greater, 300 ppm or greater, 350 ppm or greater, 400 ppm or greater, 450 ppm or greater, 500 ppm or greater, 550 ppm or greater, 600 ppm or greater, 650 ppm or greater, 700 ppm or greater, 750 ppm or greater, 800 ppm or greater, 850 ppm or greater, 900 ppm or greater, 950 ppm or greater, 1000 ppm or greater, 1100 ppm or greater, 1200 ppm or greater, 1300 ppm or greater, 1400 ppm or greater, 1500 ppm or greater, 1600 ppm or greater, 1700 ppm or greater, or 1800 ppm or greater.

The content of 2-phenylethanol contained in the coffee beans may be from 9 ppm to 2500 ppm, from 15 ppm to 2500 ppm, from 20 ppm to 2500 ppm, from 30 ppm to 2500 ppm, from 40 ppm to 2500 ppm, from 50 ppm to 2500 ppm, from 100 ppm to 2500 ppm, from 150 ppm to 2500 ppm, from 200 ppm to 2500 ppm, from 250 ppm to 2500 ppm, from 300 ppm to 2500 ppm, from 350 ppm to 2500 ppm, from 400 ppm to 2500 ppm, from 450 ppm to 2500 ppm, from 500 ppm to 2500 ppm, from 550 ppm to 2500 ppm, from 600 ppm to 2500 ppm, from 650 ppm to 2500 ppm, from 700 ppm to 2500 ppm, from 750 ppm to 2500 ppm, from 800 ppm to 2500 ppm, from 850 ppm to 2500 ppm, from 900 ppm to 2500 ppm, from 950 ppm to 2500 ppm, from 1000 ppm to 2500 ppm, from 1100 ppm to 2500 ppm, from 1200 ppm to 2500 ppm, from 1300 ppm to 2500 ppm, from 1400 ppm to 2500 ppm, from 1500 ppm to 2500 ppm, from 1600 ppm to 2500 ppm, from 1700 ppm to 2500 ppm, or from 1800 ppm to 2500 ppm.

The content of 2-phenylethanol contained in the coffee beans may be from 9 ppm to 2000 ppm, from 15 ppm to 2000 ppm, from 20 ppm to 2000 ppm, from 30 ppm to 2000 ppm, from 40 ppm to 2000 ppm, from 50 ppm to 2000 ppm, from 100 ppm to 2000 ppm, from 150 ppm to 2000 ppm, from 200 ppm to 2000 ppm, from 250 ppm to 2000 ppm, from 300 ppm to 2000 ppm, from 350 ppm to 2000 ppm, from 400 ppm to 2000 ppm, from 450 ppm to 2000 ppm, from 500 ppm to 2000 ppm, from 550 ppm to 2000 ppm, from 600 ppm to 2000 ppm, from 650 ppm to 2000 ppm, from 700 ppm to 2000 ppm, from 750 ppm to 2000 ppm, from 800 ppm to 2000 ppm, from 850 ppm to 2000 ppm, from 900 ppm to 2000 ppm, from 950 ppm to 2000 ppm, from 1000 ppm to 2000 ppm, from 1100 ppm to 2000 ppm, from 1200 ppm to 2000 ppm, from 1300 ppm to 2000 ppm, from 1400 ppm to 2000 ppm, from 1500 ppm to 2000 ppm, from 1600 ppm to 2000 ppm, from 1700 ppm to 2000 ppm, or from 1800 ppm to 2000 ppm.

The content of 2-phenylethanol contained in the coffee beans may be from 9 ppm to 1800 ppm, from 15 ppm to 1800 ppm, from 20 ppm to 1800 ppm, from 30 ppm to 1800 ppm, from 40 ppm to 1800 ppm, from 50 ppm to 1800 ppm, from 100 ppm to 1800 ppm, from 150 ppm to 1800 ppm, from 200 ppm to 1800 ppm, from 250 ppm to 1800 ppm, from 300 ppm to 1800 ppm, from 350 ppm to 1800 ppm, from 400 ppm to 1800 ppm, from 450 ppm to 1800 ppm, from 500 ppm to 1800 ppm, from 550 ppm to 1800 ppm, from 600 ppm to 1800 ppm, from 650 ppm to 1800 ppm, from 700 ppm to 1800 ppm, from 750 ppm to 1800 ppm, from 800 ppm to 1800 ppm, from 850 ppm to 1800 ppm, from 900 ppm to 1800 ppm, from 950 ppm to 1800 ppm, from 1000 ppm to 1800 ppm, from 1100 ppm to 1800 ppm, from 1200 ppm to 1800 ppm, from 1300 ppm to 1800 ppm, from 1400 ppm to 1800 ppm, from 1500 ppm to 1800 ppm, from 1600 ppm to 1800 ppm, or from 1700 ppm to 1800 ppm.

The content of 2-phenylethanol contained in the coffee beans may be from 9 ppm to 1800 ppm, from 16 ppm to 1800 ppm, from 24 ppm to 1800 ppm, from 36 ppm to 1800 ppm, from 43 ppm to 1800 ppm, from 45 ppm to 1800 ppm, from 53 ppm to 1800 ppm, from 110 ppm to 1800 ppm, from 210 ppm to 1800 ppm, from 220 ppm to 1800 ppm, from 260 ppm to 1800 ppm, from 340 ppm to 1800 ppm, from 370 ppm to 1800 ppm, from 380 ppm to 1800 ppm, from 430 ppm to 1800 ppm, from 500 ppm to 1800 ppm, from 540 ppm to 1800 ppm, from 580 ppm to 1800 ppm, from 620 ppm to 1800 ppm, from 640 ppm to 1800 ppm, from 820 ppm to 1800 ppm, from 880 ppm to 1800 ppm, from 930 ppm to 1800 ppm, from 1000 ppm to 1800 ppm, or from 1100 ppm to 1800 ppm.

In the present invention, the content of 2-phenylethanol in the coffee beans is measured according to the following measurement method.

### [Measurement Method]

1 g of coffee beans is placed in a test tube, 20 mL of water and 10 mL of diethyl ether are added, and the coffee beans are allowed to soak for 1 hour. Afterwards, 8 g of sodium chloride is added, and the mixture is shaken and centrifuged. 1 mL of the resulting diethyl ether layer is taken, to which diethyl ether is further added to make 20 mL of a liquid. The content of 2-phenylethanol in the resulting liquid is measured by gas chromatography-mass spectrometry using the following gas chromatography-mass spectrometer.

### [Gas Chromatography-Mass Spectrometer]

Model: 6890A/5973N [Agilent Technologies, Inc.]
Column: DB-WAX UI (ϕ 0.25 mm × 60 m, film thickness 0.5 µm) [Agilent Technologies, Inc.]
Injection method: Split 5:1
Temperature: 220°C at sample inlet
Column: Maintain at 60°C for 1 minute, increase temperature at 10°C/min, maintain at 240°C for 5 minutes
Gas flow rate: helium (carrier gas) 1 mL/min
Ion source temperature: 230°C
Ionization method: EI
Set mass number: m/z 91.

The coffee beans according to an aspect of the present invention contain 2-phenylethanol in an amount of 9 ppm or greater, allowing for the provision of a coffee beverage having a richer aroma than conventional products.

### Method for Producing Coffee Beans

A method for producing coffee beans containing 2-phenylethanol in an amount of 9 ppm or greater according to an aspect of the present invention includes
a step (i) of adding, to raw material beans, a yeast, and phenylalanine in an amount of 0.01 g or greater per 1 kg of the raw material beans, in which the raw material beans are coffee cherries, wet parchment with outer skin and pulp of coffee cherries removed, unroasted coffee beans, or a combination of two or more thereof; and
a step (ii) of fermenting the raw material beans with the yeast and phenylalanine added.

The method for producing coffee beans described above yields unroasted coffee beans containing 2-phenylethanol in an amount of 9 ppm or greater. Roasting (heat-drying) the unroasted coffee beans resulting from the method for producing coffee beans described above yields roasted coffee beans containing 2-phenylethanol in an amount of 9 ppm or greater.

The coffee cherries (also referred to as coffee berries) serving as the raw material beans are berries of Coffea plants. A coffee cherry contains an unroasted coffee bean (seed), pulp (a part that contains sugars and other nutrients), and outer skin. Examples of the varieties include Arabica, Robusta, and Liberica, and examples of the production areas include Brazil, Ethiopia, Vietnam, and Guatemala. In the present invention, the variety and the production area are not limited.

The wet parchment serving as the raw material beans are products after the skin and pulp are removed from the coffee cherries but before the sticky substance (mucilage) attached around the seeds are removed.

The unroasted coffee beans serving as the raw material beans are obtained by removing the sticky substance of wet parchment, drying, and selecting and threshing.

In step (i), a yeast, and phenylalanine in an amount of 0.01 g or greater per 1 kg of the raw material beans are added to the raw material beans and sufficiently mixed. The mixing may be performed using a device such as a mixer, or may be performed manually. Note that, in addition to the yeast and phenylalanine, water and/or a medium for yeast may be added to the raw material beans.

The yeast is selected from the group consisting of Candida sake, Brettanomyces sp., Zygosaccharomyces bisporus, Saccharomyces cerevisiae, and a combination of two or more of the foregoing.

In step (i), a medium suitable for yeast may be further added. In particular, when unroasted coffee beans are used as the raw material beans, it is preferable to add a medium that resembles the sticky substance (mucilage). Such a medium may be, but is not limited to, a medium obtained by dissolving and mixing from 20 to 60 g of sucrose, from 15 to 45 g of glucose, from 30 to 90 g of pectin, and from 15 to 45 g of fructose per 1 liter (L) of a medium. The resulting medium further added with glucose may also be used as the medium. The amount of the medium to be added may be 1 mL or greater, 5 mL or greater, 10 mL or greater, 30 mL or greater, 50 mL or greater, 100 mL or greater, from 1 to 100 mL, from 5 to 100 mL, from 10 to 100 mL, from 30 to 100 mL, or from 50 to 100 mL per 100 g of the raw material beans.

The amount of phenylalanine added in step (i) may be 0.2 g or greater, 0.3 g or greater, 0.4 g or greater, 0.5 g or greater, 0.75 g or greater, 1.0 g or greater, 1.5 g or greater, 2.0 g or greater, 3.0 g or greater, 4.0 g or greater, 5.0 g or greater, 6.0 g or greater, 8.0 g or greater, 10 g or greater, 12 g or greater, 16 g or greater, 20 g or greater, 24 g or greater, 30 g or greater, 36 g or greater, 40 g or greater, 48 g or greater, 50 g or greater, 60 g or greater, 80 g or greater, or 100 g or greater per 1 kg of the raw material beans.

The amount of phenylalanine added in step (i) may be from 0.01 g to 200 g, from 0.01 g to 150 g, from 0.01 g to 100 g, from 0.01 g to 80 g, from 0.01 g to 60 g, from 0.01 g to 50 g, from 0.01 g to 48 g, from 0.01 g to 40 g, from 0.01 g to 40 g, from 0.01 g to 36 g, from 0.01 g to 30 g, from 0.01 g to 24 g, from 0.01 g to 20 g, from 0.01 g to 16 g, from 0.01 g to 12 g, from 0.01 g to 10 g, from 0.01 g to 8.0 g, from 0.05 g to 200 g, from 0.05 g to 150 g, from 0.05 g to 100 g, from 0.05 g to 80 g, from 0.05 g to 60 g, from 0.05 g to 50 g, from 0.05 g to 48 g, from 0.05 g to 40 g, from 0.05 g to 40 g, from 0.05 g to 36 g, from 0.05 g to 30 g, from 0.05 g to 24 g, from 0.05 g to 20 g, from 0.05 g to 16 g, from 0.05 g to 12 g, from 0.05 g to 10 g, from 0.05 g to 8.0 g, from 0.1 g to 200 g, from 0.1 g to 150 g, from 0.1 g to 100 g, from 0.1 g to 80 g, from 0.1 g to 60 g, from 0.1 g to 50 g, from 0.1 g to 48 g, from 0.1 g to 40 g, from 0.1 g to 40 g, from 0.1 g to 36 g, from 0.1 g to 30 g, from 0.1 g to 24 g, from 0.1 g to 20 g, from 0.1 g to 16 g, from 0.1 g to 12 g, from 0.1 g to 10 g, from 0.1 g to 8.0 g, from 0.4 g to 200 g, from 0.4 g to 150 g, from 0.4 g to 100 g, from 0.4 g to 80 g, from 0.4 g to 60 g, from 0.4 g to 50 g, 0.4 g to 48 g, from 0.4 g to 40 g, from 0.4 g to 40 g, from 0.4 g to 36 g, from 0.4 g to 30 g, from 0.4 g to 24 g, from 0.4 g to 20 g, from 0.4 g to 16 g, from 0.4 g to 12 g, from 0.4 g to 10 g, from 0.4 g to 8.0 g, from 0.75 g to 200 g, from 0.75 g to 150 g, from 0.75 g to 100 g, from 0.75 g to 80 g, from 0.75 g to 60 g, from 0.75 g to 50 g, from 0.75 g to 48 g, from 0.75 g to 40 g, from 0.75 g to 40 g, from 0.75 g to 36 g, 0.75 g to 30 g, from 0.75 g to 24 g, from 0.75 g to 20 g, from 0.75 g to 16 g, from 0.75 g to 12 g, from 0.75 g to 10 g, from 0.75 g to 8.0 g, from 1.0 g to 200 g, from 1.0 g to 150 g, from 1.0 g to 100 g, from 1.0 g to 80 g, from 1.0 g to 60 g, from 1.0 g to 50 g, from 1.0 g to 48 g, from 1.0 g to 40 g, from 1.0 g to 40 g, from 1.0 g to 36 g, from 1.0 g to 30 g, from 1.0 g to 24 g, from 1.0 g to 20 g, from 1.0 g to 16 g, from 1.0 g to 12 g, from 1.0 g to 10 g, from 1.0 g to 8.0 g, from 5.0 g to 200 g, from 5.0 g to 150 g, from 5.0 g to 5.00 g, from 5.0 g to 80 g, from 5.0 g to 60 g, from 5.0 g to 50 g, from 5.0 g to 48 g, from 5.0 g to 40 g, from 5.0 g to 40 g, from 5.0 g to 36 g, from 5.0 g to 30 g, from 5.0 g to 24 g, from 5.0 g to 20 g, from 5.0 g to 16 g, from 5.0 g to 12 g, or from 5.0 g to 8.0 g per 1 kg of the raw material beans.

In step (ii), the raw material beans mixed with yeast and phenylalanine are placed in a container or a bag and then fermented for 1 hour or longer. The fermentation duration may be 6 hours or longer, 12 hours or longer, 24 hours or longer, 36 hours or longer, 48 hours or longer, 60 hours or longer, 72 hours or longer, 90 hours or longer, 100 hours or longer, from 1 to 100 hours, from 1 to 90 hours, from 1 to 72 hours, from 1 to 60 hours, from 1 to 48 hours, from 1 to 36 hours, from 1 to 24 hours, from 1 to 12 hours, from 1 to 6 hours, from 6 to 100 hours, from 6 to 90 hours, from 6 to 72 hours, from 6 to 60 hours, from 6 to 48 hours, from 6 to 36 hours, from 6 to 24 hours, from 6 to 12 hours, from 12 to 100 hours, from 12 to 90 hours, from 12 to 72 hours, from 12 to 60 hours, from 12 to 48 hours, from 12 to 36 hours, from 12 to 24 hours, from 24 to 100 hours, from 24 to 90 hours, from 24 to 72 hours, from 24 to 60 hours, from 24 to 48 hours, or from 24 to 36 hours.

The fermentation in step (ii) is not limited as long as the conditions allow fermentation to be carried out, and conditions suitable for fermentation (for example, the type and amount of yeast to be used, the type and amount of nutritive substances, temperature, humidity, pH, oxygen concentration, carbon dioxide concentration, and/or fermentation duration) may be appropriately set as necessary. The method for producing coffee beans may be carried out under the atmospheric temperature and atmospheric humidity of the place where it is carried out, or may be carried out in an environment where the temperature and humidity are controlled, such as in a laboratory or factory. Fermentation may be carried out at a controlled temperature ranging from 5°C to 100°C (for example, from 20 to 40°C, from 22 to 34°C, or from 24 to 30°C) and a controlled humidity ranging from 5% to 100% (for example, from 30 to 90%, from 40 to 80%, or from 50 to 70%), although the fermentation is not limited by the foregoing. The fermentation duration, temperature, and humidity may be appropriately adjusted depending on the type of yeast or raw material beans to be used. The fermentation is carried out under atmospheric pressure, but may also be carried out under reduced pressure or increased pressure.

Examples of the nutritive substances used in the fermentation in step (ii) include pulp, fruit juice, sugars, and a medium. The pulp is, for example, coffee pulp (a sticky substance also known as mucilage), and may be undried or dried. Note that not only coffee pulp but also other types of pulp such as grape pulp, cherry pulp, and peach pulp, or any combination thereof may be used. The nutritive substances other than pulp may be any nutritive substances that can be assimilated by yeast, such as fruit juice (such as that of grape, peach, and apple), sugars (e.g., monosaccharides, disaccharides, and polysaccharides obtained from plants such as sugarcane and sweet potato), grains (e.g., wort made by saccharifying malt), and medium.

The amount of yeast used in an embodiment of the present invention is not limited as long as the effect of increasing the 2-phenylethanol content can be achieved. For example, the yeast may be added in an amount of from 1.0 × 10⁴ cells/g to 1.0 × 10¹⁴ cells/g, from 1.0 × 10⁶ cells/g to 1.0 × 10¹² cells/g, or from 1.0 × 10⁸ cells/g to 1.0 × 10¹⁰ cells/g per weight of coffee beans.

When finishing the fermentation process, heat sterilization, water washing, drying, roasting, or the like may be carried out in any combination. When a dryer is used in the drying, the fermentation may be finished with drying at any temperature (for example, from 20 to 70°C, from 30 to 70°C, from 40 to 70°C, or from 50 to 70°C) for approximately from 1 to 3 days. Note that, the fermentation may be carried out in accordance with the fermentation described in the prior patent JP 5032979 B by the applicant.

The method for producing coffee beans may further include a step (iii) of drying the raw material beans fermented in the step (ii). Also, the method for producing coffee beans may further include a step (iv) of selecting and threshing the raw material beans dried in the step (iii) to produce coffee beans. In addition, the method for producing coffee beans further includes, as necessary, a step (v) of roasting (heat-drying) the beans already subjected to the step (iii) of drying and the step (iv) of selecting and threshing to produce roasted coffee beans.

The method for producing coffee beans can be carried out in a country where coffee cherries are produced or in a country where coffee beans are consumed. In addition, the method for producing coffee beans may be carried out in a ship or the like during the transportation of coffee beans.

### Examples

### Examples 1 to 6

In Examples 1 to 6 and Comparative Examples 1 to 4 of the present invention, the raw material beans used were wet parchment (WP) with a sticky substance (mucilage) attached around the seeds. The wet parchment was obtained by removing skin and pulp from coffee cherries produced in a farm in Brazil (referred to as farm A). In addition, the production of unroasted coffee beans and roasted coffee beans using the raw material beans was carried out in the farm A.

In each of Examples 1 to 6, a yeast Saccharomyces cerevisiae and phenylalanine in the amount presented in Table 1 were added to the wet parchment serving as the raw material beans, and mixed. The mixture was placed in a plastic bag and allowed to stand. In each of Comparative Examples 1 to 4, a yeast Saccharomyces cerevisiae was added to the wet parchment serving as the raw material beans, and mixed. The mixture was placed in a plastic bag and allowed to stand. The amount of yeast added was the same value (1.0 × 10⁷ cells/g) in each of Examples 1 to 6 and Comparative Examples 1 to 4.

The raw material beans in the plastic bag to which the yeast and phenylalanine were added were fermented for a duration presented in Table 1. This fermentation was performed under the atmospheric pressure, the atmospheric temperature, and the atmospheric humidity at the location of the farm A, and the conditions were the same in all of Examples 1 to 6 and Comparative Examples 1 to 4 except for the fermentation duration.

After the fermentation, the fermented raw material beans were subjected to drying as well as selecting and threshing, resulting in unroasted coffee beans.

The unroasted coffee beans were subjected to roasting, resulting in roasted coffee beans.

Drying, selecting and threshing, and roasting were performed under the same conditions in all of Examples 1 to 6 and Comparative Examples 1 to 4.

The contents of 2-phenylethanol contained in the unroasted coffee beans and the roasted coffee beans were measured by the following method.

### [Measurement Method of 2-Phenylethanol Concentration (Content)]

1 g of coffee beans (unroasted coffee beans or roasted coffee beans) is placed in a test tube, 20 mL of water and 10 mL of diethyl ether are added, and the coffee beans are allowed to soak for 1 hour. Afterwards, 8 g of sodium chloride is added, and the mixture is shaken and centrifuged. 1 mL of the resulting diethyl ether layer is taken, to which diethyl ether is further added to make 20 mL of a liquid. The content of 2-phenylethanol in the resulting liquid is measured by gas chromatography-mass spectrometry using the following gas chromatography-mass spectrometer.

### [Gas Chromatography-Mass Spectrometer]

Model: 6890A/5973N [Agilent Technologies, Inc.]
Column: DB-WAX UI (ϕ 0.25 mm × 60 m, film thickness 0.5 µm) [Agilent Technologies, Inc.]
Injection method: Split 5:1
Temperature: 220°C at sample inlet
Column: Maintain at 60°C for 1 minute, increase temperature at 10°C/min, maintain at 240°C for 5 minutes
Gas flow rate: helium (carrier gas) 1 mL/min
Ion source temperature: 230°C
Ionization method: EI
Set mass number: m/z 91

The content of 2-phenylethanol in the unroasted coffee beans and the content of 2-phenylethanol in the roasted coffee beans in each of Examples 1 to 6 and Comparative Examples 1 to 4 were measured, and the results are presented in Table 1.

**Table 1**

| | Raw Material Beans | Yeast | Phenylalanine Addition Amount (g/1 kg of raw material beans) | Fermentation Duration (hours) | 2-phenylethanol Concentration in Unroasted Coffee Beans (ppm) | 2-phenylethanol Concentration in Roasted Coffee Beans (ppm) |
|---|---|---|---|---|---|---|
| Example 1 | WP | *Saccharomyces cerevisiae* | 1 | 24 | 210 | 240 |
| Example 2 | WP | *Saccharomyces cerevisiae* | 1 | 48 | 260 | 320 |
| Example 3 | WP | *Saccharomyces cerevisiae* | 1 | 72 | 370 | 390 |
| Example 4 | WP | *Saccharomyces cerevisiae* | 3 | 24 | 380 | 440 |
| Example 5 | WP | *Saccharomyces cerevisiae* | 3 | 48 | 620 | 630 |
| Example 6 | WP | *Saccharomyces cerevisiae* | 3 | 72 | 580 | 600 |
| Comparative Example 1 | WP | *Saccharomyces cerevisiae* | 0 | 0 | 3.7 | 2.5 |
| Comparative Example 2 | WP | *Saccharomyces cerevisiae* | 0 | 24 | 7.6 | 1.6 |
| Comparative Example 3 | WP | *Saccharomyces cerevisiae* | 0 | 48 | 4.2 | 6.3 |
| Comparative Example 4 | WP | *Saccharomyces cerevisiae* | 0 | 72 | 4.0 | 5.7 |

As presented in Table 1, in the unroasted coffee beans according to Examples 1 to 6, the concentrations (contents) of 2-phenylethanol were from 210 to 620 ppm. Meanwhile, in the unroasted coffee beans according to Comparative Examples 1 to 4, the contents of 2-phenylethanol were from 3.7 to 7.6 ppm. Comparing the two, the contents of 2-phenylethanol in the unroasted coffee beans according to Examples 1 to 6 increased significantly by about 28 to 168 times that of the unroasted coffee beans of Comparative Examples 1 to 4.

In the roasted coffee beans according to Examples 1 to 6, the concentrations (contents) of 2-phenylethanol were from 240 to 630 ppm. Meanwhile, in the unroasted coffee beans according to Comparative Examples 1 to 4, the contents of 2-phenylethanol were from 2.5 to 25 ppm. Comparing the two, the contents of 2-phenylethanol in the roasted coffee beans according to Examples 1 to 6 increased significantly by about 10 to 252 times that of the roasted coffee beans of Comparative Examples 1 to 4. Furthermore, the contents of 2-phenylethanol in the roasted coffee beans of Examples 1 to 6 increased by about 1.02 to 1.23 times that of the unroasted coffee beans of Examples 1 to 6.

As indicated by these examples, the unroasted coffee beans of Examples 1 to 6 contain significantly increased amounts of 2-phenylethanol as compared with the unroasted coffee beans of Comparative Examples 1 to 4. Also, the roasted coffee beans contain increased amounts of 2-phenylethanol as compared with the unroasted coffee beans.

### Examples 7 to 12

In Examples 7 to 12 and Comparative Examples 5 to 8 of the present invention, the raw material beans used were coffee cherries (CC) produced in another farm in Brazil (referred to as farm B) and wet parchment (WP) that has a sticky substance (mucilage) attached around the seeds and that results from removing skin and pulp from the coffee cherries. In addition, the production of unroasted coffee beans using the raw material beans was carried out in the farm B.

In each of Examples 7 to 9, a yeast Saccharomyces cerevisiae and phenylalanine in the amount presented in Table 2 were added to the wet parchment serving as the raw material beans, and mixed. The mixture was placed in a plastic bag and allowed to stand.

In each of Examples 10 to 12, a yeast Saccharomyces cerevisiae and phenylalanine in the amount presented in Table 2 were added to the coffee cherries serving as the raw material beans, and mixed. The mixture was placed in a plastic bag and allowed to stand.

In each of Comparative Examples 5 to 8, a yeast Saccharomyces cerevisiae was added to the wet parchment serving as the raw material beans, and mixed. The mixture was placed in a plastic bag and allowed to stand.

The amount of yeast added was the same value (1.0 × 10⁷ cells/g) in each of Examples 7 to 12 and Comparative Examples 5 to 8.

The raw material beans in the plastic bag to which the yeast and phenylalanine were added were fermented for a duration presented in Table 2. This fermentation was performed under the atmospheric pressure, the atmospheric temperature, and the atmospheric humidity at the location of the farm B, and the conditions were the same in all of Examples 7 to 12 and Comparative Examples 5 to 8 except for the fermentation duration.

After the fermentation, the fermented raw material beans were subjected to drying as well as selecting and threshing, resulting in unroasted coffee beans. Drying as well as selecting and threshing were performed under the same conditions in all of Examples 7 to 12 and Comparative Examples 5 to 8.

The contents of 2-phenylethanol contained in the unroasted coffee beans were measured by the measurement method of 2-phenylethanol concentration (content) described above. The contents of 2-phenylethanol were measured for the unroasted coffee beans in each of Examples 7 to 12 and Comparative Examples 5 to 8, and the results are presented in Table 2.

**[Table 2]**

| | Raw Material Beans | Yeast | Phenylalanine Addition Amount (g/1 kg of raw material beans) | Fermentation Duration (hours) | 2-phenylethanol Concentration in Unroasted Coffee Beans (ppm) |
|---|---|---|---|---|---|
| Example 7 | WP | *Saccharomyces cerevisiae* | 0.75 | 24 | 24 |
| Example 8 | WP | *Saccharomyces cerevisiae* | 0.75 | 48 | 43 |
| Example 9 | WP | *Saccharomyces cerevisiae* | 0.75 | 72 | 110 |
| Example 10 | CC | *Saccharomyces cerevisiae* | 0.4 | 24 | 9 |
| Example 11 | CC | *Saccharomyces cerevisiae* | 0.4 | 48 | 16 |
| Example 12 | CC | *Saccharomyces cerevisiae* | 0.4 | 72 | 36 |
| Comparative Example 5 | WP | *Saccharomyces cerevisiae* | 0 | 0 | 0.3 |
| Comparative Example 6 | WP | *Saccharomyces cerevisiae* | 0 | 24 | 0.2 |
| Comparative Example 7 | WP | *Saccharomyces cerevisiae* | 0 | 48 | 0.3 |
| Comparative Example 8 | WP | *Saccharomyces cerevisiae* | 0 | 72 | 0.5 |

As presented in Table 2, in the unroasted coffee beans according to Examples 7 to 9 in which wet parchment was used as the raw material beans, the concentrations (contents) of 2-phenylethanol were from 24 to 110 ppm. In the unroasted coffee beans according to Examples 10 to 12 in which coffee cherries were used as raw material beans, the contents of 2-phenylethanol were from 9 to 36 ppm. Meanwhile, in the unroasted coffee beans according to Comparative Examples 5 to 8, the contents of 2-phenylethanol were from 0.2 to 0.5 ppm. Comparing Examples and Comparative Examples, the contents of 2-phenylethanol in the unroasted coffee beans according to Examples 7 to 12 increased significantly by about 28 to 550 times that of the unroasted coffee beans of Comparative Examples 5 to 6.

As described above, the unroasted coffee beans of Examples 7 to 12 contained significantly increased 2-phenylethanol contents as compared with the unroasted coffee beans of Comparative Examples 5 to 8. Moreover, by turning the unroasted coffee beans of Examples 7 to 12 into roasted coffee beans, the contents of 2-phenylethanol can further increase as compared with the unroasted coffee beans.

### Examples 13 to 30

In Examples 13 to 30 and Comparative Examples 9 and 10 of the present invention, the raw material beans used were unroasted coffee beans (GB). The unroasted coffee beans (GB) were made from coffee cherries (CC) grown in a farm in Brazil. The unroasted coffee beans (GB) were produced in the same farm and then imported to Japan. The production of unroasted coffee beans according to Examples 13 to 30 and Comparative Examples 9 to 10 were carried out in a laboratory in Japan.

A basal medium resembling the sugar composition of mucilage was prepared and used as the medium (see "Coffee - Growing, Processing, Sustainable Production: A Guidebook for Growers, Processors, Traders and Researchers (Paperback)"). The basal medium was prepared by dissolving 40 g of sucrose, 30 g of glucose, 66 g of pectin, and 30 g of fructose per 1 liter (L) of water. In Examples 14 to 21, a medium obtained by further adding glucose (D-glucopyranose) to the basal medium was used as the medium.

In Example 13, a yeast Saccharomyces cerevisiae, phenylalanine in the amount presented in Table 3 (6 g per 1 kg of raw material beans), and the basal medium (50 mL per 100 g of raw material beans) were added to the unroasted coffee beans (GB) serving as the raw material beans, and mixed. The mixture was placed in a plastic bag and allowed to stand.

In Example 14, a yeast Saccharomyces cerevisiae, phenylalanine in the amount presented in Table 3 (6 g per 1 kg of raw material beans), the basal medium (50 mL per 100 g of raw material beans), and glucose (1 g per 100 g of raw material beans) were added to the unroasted coffee beans (GB) serving as the raw material beans, and mixed. The mixture was placed in a plastic bag and allowed to stand.

Similarly, in each of Examples 15 to 30, a yeast Saccharomyces cerevisiae, Candida sake, Zygosaccharomyces bisporus, or Brettanomyces sp., phenylalanine in the amount presented in Table 3, and a medium were added to unroasted coffee beans (GB) serving as the raw material beans, and mixed. The mixture was placed in a plastic bag and allowed to stand.

In each of Comparative Examples 9 and 10, a yeast Saccharomyces cerevisiae and a medium were added to unroasted coffee beans (GB) serving as the raw material beans, and mixed. The mixture was placed in a plastic bag and allowed to stand.

The amount of yeast added was the same value (1.0 × 10⁴ cells/g) in each of Examples 13 to 30 and Comparative Examples 9 to 10.

The raw material beans in the plastic bag to which the yeast, phenylalanine, and the medium were added were fermented for the duration presented in Table 3. This fermentation was performed at a controlled temperature of 28°C and under atmospheric pressure and atmospheric humidity in a laboratory in Japan. The conditions were the same in all of Examples 13 to 30 and Comparative Examples 9 to 10 except for the fermentation duration.

After the fermentation, the fermented raw material beans were subjected to drying, resulting in unroasted coffee beans. Drying was performed under the same conditions in all of Examples 13 to 30 and Comparative Examples 9 to 10.

The contents of 2-phenylethanol contained in the unroasted coffee beans were measured by the measurement method of 2-phenylethanol concentration (content) described above. The contents of 2-phenylethanol were measured for the unroasted coffee beans in each of Examples 13 to 30 and Comparative Examples 9 to 10, and the results are presented in Table 3.

**[Table 3]**

| | Raw Material Beans | Yeast | Phenylalanine Addition Amount (g/1 kg of raw material beans) | Medium (amount per 100 g of raw material beans) | Fermentation Duration (hours) | 2-phenylethanol Concentration in Unroasted Coffee Beans (ppm) |
|---|---|---|---|---|---|---|
| Example 13 | GB | *Saccharomyces cerevisiae* | 6 | Basal Medium 50 mL/100 g | 24 | 880 |
| Example 14 | GB | *Saccharomyces cerevisiae* | 6 | Basal Medium 10 mL/100 g + Glucose 1 g/100 g | 24 | 540 |
| Example 15 | GB | *Saccharomyces cerevisiae* | 6 | Basal Medium 10 mL/100 g + Glucose 2 g/100 g | 24 | 500 |
| Example 16 | GB | *Saccharomyces cerevisiae* | 6 | Basal Medium 10 mL/100 g + Glucose 4 g/100 g | 24 | 540 |
| Example 17 | GB | *Saccharomyces cerevisiae* | 6 | Basal Medium 10 mL/100 g + Glucose 10 g/100 g | 24 | 430 |
| Example 18 | GB | *Saccharomyces cerevisiae* | 6 | Basal Medium 50 mL/100 g | 24 | 930 |
| Example 19 | GB | *Saccharomyces cerevisiae* | 6 | Basal Medium 10 mL/100 g + Glucose 1 g/100 g | 24 | 640 |
| Example 20 | GB | *Saccharomyces cerevisiae* | 6 | Basal Medium 10 mL/100 g + Glucose 1 g/100 g | 48 | 820 |
| Example 21 | GB | *Saccharomyces cerevisiae* | 6 | Basal Medium 10 mL/100 g + Glucose 1 g/100 g | 72 | 1100 |
| Example 22 | GB | *Saccharomyces cerevisiae* | 6 | Basal Medium 50 mL/100 g | 48 | 1000 |
| Example 23 | GB | *Candida sake* | 6 | Basal Medium 50 mL/100 g | 48 | 45 |
| Example 24 | GB | *Izygosaccharomyces bisporus* | 6 | Basal Medium 50 mL/100 g | 48 | 340 |
| Example 25 | GB | *Brettanomyces sp.* | 6 | Basal Medium 50 mL/100 g | 48 | 53 |
| Example 26 | GB | *Saccharomyces cerevisiae* | 12 | Basal Medium 50 mL/100 g | 48 | 1500 |
| Example 27 | GB | *Saccharomyces cerevisiae* | 24 | Basal Medium 50 mL/100 g | 48 | 1800 |
| Example 28 | GB | *Saccharomyces cerevisiae* | 36 | Basal Medium 50 mL/100 g | 48 | 1700 |
| Example 29 | GB | *Saccharomyces cerevisiae* | 48 | Basal Medium 50 mL/100 g | 48 | 1700 |
| Example 30 | GB | *Saccharomyces cerevisiae* | 60 | Basal Medium 50 mL/100 g | 48 | 1500 |
| Comparative Example 9 | GB | *Saccharomyces cerevisiae* | 0 | Basal Medium 50 mL/100 g | 24 | 0.3 |
| Comparative Example 10 | GB | *Saccharomyces cerevisiae* | 0 | Basal Medium 50 mL/100 g | 48 | 0.9 |

As presented in Table 3, in the unroasted coffee beans according to Examples 13 to 30, the concentrations (contents) of 2-phenylethanol were from 45 to 1800 ppm. Meanwhile, in the unroasted coffee beans according to Comparative Examples 9 to 10, the contents of 2-phenylethanol were from 0.3 to 0.9 ppm. Comparing Examples and Comparative Examples, the contents of 2-phenylethanol in the unroasted coffee beans according to Examples 13 to 30 increased significantly by about 50 to 6000 times that of the unroasted coffee beans of Comparative Examples 9 to 10.

As described above, the unroasted coffee beans of Examples 13 to 30 contain significantly increased amounts of 2-phenylethanol as compared with the unroasted coffee beans of Comparative Examples 9 to 10.

### Comparative Examples 11 to 19

In Comparative Examples 11 to 19, commercially available unroasted coffee beans were purchased, and the contents of 2-phenylethanol contained in the commercially available unroasted coffee beans were measured by the above-described measurement method.

**[Table 4]**

| | Product Name of Unroasted Coffee Beans | 2-Phenylethanol Concentration in Unroasted Coffee Beans (ppm) |
|---|---|---|
| Comparative Example 11 | Guatemala SHB | 1.8 |
| Comparative Example 12 | Columbia Supremo | 0.5 |
| Comparative Example 13 | Ethiopia Guji | 1.5 |
| Comparative Example 14 | Vietnam Robusta | 2 |
| Comparative Example 15 | Columbia Huila Area | 0.3 |
| Comparative Example 16 | Columbia Alto Del Obispo La Esperanza Farm (Caturra) | 0.3 |
| Comparative Example 17 | Indonesia Aceh Alurbadak | 0.5 |
| Comparative Example 18 | Costa Rica Herbazu Leoncio Black Honey | 0.3 |
| Comparative Example 19 | Kenya Kariaini AB | 0.3 |

The concentrations (contents) of 2-phenylethanol in the commercially available unroasted coffee beans according to Comparative Examples 11 to 19 were from 0.3 to 2 ppm.

## Claims

1. Coffee beans comprising 2-phenylethanol in an amount of 9 ppm or greater.

2. The coffee beans according to claim 1, wherein the coffee beans contain the 2-phenylethanol in an amount from 9 ppm to 2000 ppm.

3. The coffee beans according to claim 1 or 2, wherein the coffee beans are unroasted coffee beans or roasted coffee beans.

4. The coffee beans according to any one of claims 1 to 3, wherein the content of 2-phenylethanol in the coffee beans is measured according to the following measurement method:
[Measurement Method]
1 g of coffee beans is placed in a test tube, 20 mL of water and 10 mL of diethyl ether are added, and the coffee beans are allowed to soak for 1 hour; afterwards, 8 g of sodium chloride is added, and the mixture is shaken and centrifuged; 1 mL of the resulting diethyl ether layer is taken, to which diethyl ether is further added to make 20 mL of a liquid; the content of 2-phenylethanol in the resulting liquid is measured by gas chromatography-mass spectrometry using the following gas chromatography-mass spectrometer:
[Gas Chromatography-Mass Spectrometer]
Model: 6890A/5973N [Agilent Technologies, Inc.]
Column: DB-WAX UI (ϕ 0.25 mm × 60 m, film thickness 0.5 µm) [Agilent
Technologies, Inc.]
Injection method: Split 5:1
Temperature: 220°C at sample inlet
Column: Maintain at 60°C for 1 minute, increase temperature at 10°C/min, maintain at 240°C for 5 minutes
Gas flow rate: helium (carrier gas) 1 mL/min
Ion source temperature: 230°C
Ionization method: EI
Set mass number: m/z 91.

5. A method for producing coffee beans containing 2-phenylethanol in an amount of 9 ppm or greater, the method comprising
(i) adding, to raw material beans, a yeast, and phenylalanine in an amount of 0.01 g or greater per 1 kg of the raw material beans, in which the raw material beans are coffee cherries, wet parchment with outer skin and pulp of coffee cherries removed, unroasted coffee beans, or a combination of two or more thereof; and
(ii) fermenting the raw material beans with the yeast and phenylalanine added.

6. The production method according to claim 5, wherein the yeast is selected from the group consisting of Candida sake, Brettanomyces sp., Zygosaccharomyces bisporus, Saccharomyces cerevisiae, and a combination of two or more of the foregoing.

7. The production method according to claim 5 or 6, wherein in the step (ii), the raw material beans are fermented for 1 hour or longer.

8. The method for producing coffee beans according to any one of claims 5 to 7, wherein a content of 2-phenylethanol in the coffee beans is measured according to the following measurement method:
[Measurement Method]
1 g of coffee beans is placed in a test tube, 20 mL of water and 10 mL of diethyl ether are added, and the coffee beans are allowed to soak for 1 hour; afterwards, 8 g of sodium chloride is added, and the mixture is shaken and centrifuged; 1 mL of the resulting diethyl ether layer is taken, to which diethyl ether is further added to make 20 mL of a liquid; the content of 2-phenylethanol in the resulting liquid is measured by gas chromatography-mass spectrometry using the following gas chromatography-mass spectrometer;
[Gas Chromatography-Mass Spectrometer]
Model: 6890A/5973N [Agilent Technologies, Inc.]
Column: DB-WAX UI (ϕ 0.25 mm × 60 m, film thickness 0.5 µm) [Agilent
Technologies, Inc.]
Injection method: Split 5:1
Temperature: 220°C at sample inlet
Column: Maintain at 60°C for 1 minute, increase temperature at 10°C/min, maintain at 240°C for 5 minutes
Gas flow rate: helium (carrier gas) 1 mL/min
Ion source temperature: 230°C
Ionization method: EI
Set mass number: m/z 91.
